Europäisches Patentamt

European Patent Office

Office européen des brevets

(19)

(11) **EP 1 598 432 A2**

(12) **EUROPEAN PATENT APPLICATION**

(43) Date of publication:
23.11.2005 Bulletin 2005/47

(51) Int Cl.$^7$: **C12Q 1/68**

(21) Application number: **05016660.2**

(22) Date of filing: **31.07.2001**

(84) Designated Contracting States:
**AT BE CH CY DE DK ES FI FR GB GR IE IT LI LU MC NL PT SE TR**

(30) Priority: **31.07.2000 US 628472**

(62) Document number(s) of the earlier application(s) in accordance with Art. 76 EPC:
**01306573.5 / 1 180 548**

(71) Applicant: **Agilent Technologies, Inc.**
**Palo Alto, CA 94306 (US)**

(72) Inventors:
• **Wolber, Paul K.**
**Los Altos, CA 94022 (US)**
• **Amorese, Douglas A.**
**Los Altos, CA 94022 (US)**
• **Kincaid, Robert H.**
**Half Moon Bay, CA 94019 (US)**
• **Ilsley, Diane D.**
**San Jose, CA 95123 (US)**
• **Atwell, Andrew S.**
**Sunnyvale, CA 94086 (US)**

(74) Representative: **Tollett, Ian et al**
**Williams Powell**
**Morley House**
**26-30 Holborn Viaduct**
**London EC1A 2BP (GB)**

Remarks:
This application was filed on 01.08.2005 as a divisional application to the application mentioned under INID code 62.

(54) **Array based methods for sythesizing nucleic acid mixtures**

(57) Methods for generating mixtures of nucleic acids, e.g., oligonucleotide primers, are provided. In the subject methods, an array is employed as template to generate mixtures of nucleic acids via a template driven primer extension reaction. In preferred embodiments, each probe on the array employed in the subject methods comprises a constant domain and a variable domain, where the constant domain is further characterized by having at least a recognition domain. Also provided are the arrays employed in the subject methods and kits for practicing the subject methods. The subject methods find use in a variety of applications, including the generation of target nucleic acids from an mRNA sample for use in hybridization assays, e.g., differential gene expression analyses.

FIGURE 1

L= 77KB RNA ladder
1= Experimental array 2 (annealed with PT7)
2= Concentrated experimental array 2
3= Positive control array 4 (annealed with PT7 AND PT7/HVC185 complex in mix)
4= Concentrated positive control array 4
5= Positive control tube

EP 1 598 432 A2

**Description**

The present invention relates to a method for producing a mixture of nucleic acids, and in particular oligonucleotide primers. The general field of this invention is molecular biology, and particularly gene expression analysis.

The characterization of cellular gene expression (i.e., gene expression analysis) finds application in a variety of disciplines, such as in the analysis of differential expression between different tissue types, different stages of cellular growth or between normal and diseased states.

Fundamental to differential expression analysis is the detection of different mRNA species in a test sample, and often the quantitative determination of different mRNA levels in that test sample. In order to detect different mRNA levels in a given test population, a population of labeled target nucleic acids that, at least partially, reflects or mirrors the mRNA profile of the test sample is produced. In other words, a population of labeled target nucleic acids is generated where at least a portion of the mRNA species in the test sample are represented, in terms of presence and often in terms of amount. Following target generation, the target population is contacted with one or more probe sequences, e.g., as found on an array, whereby the presence and often amount of specific targets in the target population is detected. From the resultant data, information about the mRNAs present in the sample, i.e., the mRNA profile and gene expression profile, can be readily deduced.

A fundamental step in gene expression analysis assays is, therefore, the step of labeled target generation. Target generation protocols typically include a primer extension reaction, in which a primer is contacted with an initial mRNA sample to produce a labeled target population, as described above. In certain protocols, polyA primers and variants thereof are employed. Disadvantages of such protocols include the inability to produce target from prokaryotic mRNA species that lack a polyA tail and the propensity of such protocols to produce target that lacks 5' mRNA information. While the use of random primers overcomes some of these disadvantages, random primer protocols suffer from their own disadvantages, e.g., lack of specificity resulting from increased complexity in the primer mixture produced by the process, where not only mRNA is represented, but also rRNA, tRNA and snRNA. In yet other protocols, custom primer mixes are employed in target generation. While such protocols overcome the above-described disadvantages with polyA and random primer based protocols, custom primer mix or gene specific primer based protocols can be prohibitively expensive, particularly in array-based hybridization protocols in which custom arrays are employed.

As such, there is continued interest in the development of new primer generation protocols. Of particular interest would be the development of a protocol that realizes the advantages of gene specific primer based protocols while at the same time is economical to perform and is therefore suitable for use in custom array-based hybridization assays.

Relevant Literature

See U.S. Patent No. 5,795,714 and the references cited therein.

Methods for generating mixtures of nucleic acids, e.g., oligonucleotide primers, are provided. In the subject methods, an array of probe nucleic acids is employed as template to generate mixtures of nucleic acids via a template driven primer extension reaction. In preferred embodiments, each probe on the array employed in the subject methods comprises a constant domain and a variable domain, where the constant domain is further characterized by having at least a recognition domain, and optionally a functional domain and/or linker domain. Also provided are the arrays employed in the subject methods and kits for practicing the subject methods. The subject methods find use in a variety of applications, including the generation of target nucleic acids from an mRNA sample for use in hybridization assays, e.g., differential gene expression analysis.

A number of preferred embodiments of the present invention will now be disclosed, with reference to the drawings, in which: -

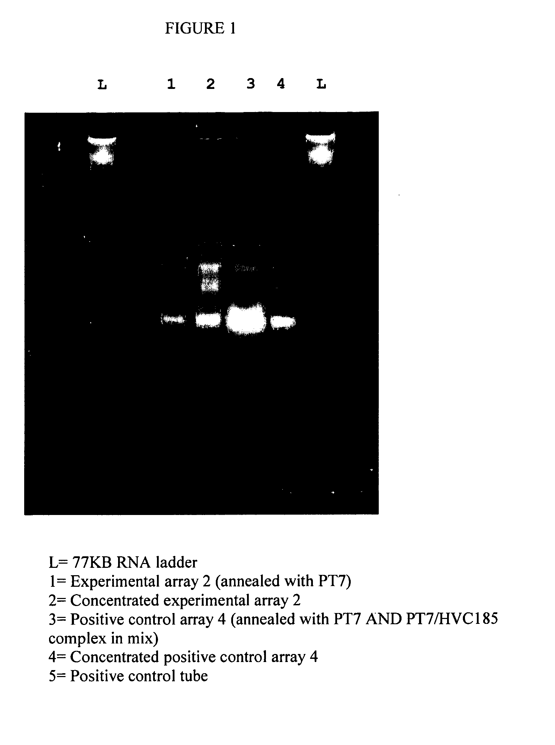

Figure 1 provides a view of the stained gel produced in Example 1 of the Experimental section, infra.

The term "nucleic acid" as used herein means a polymer composed of nucleotides, e.g., deoxyribonucleotides or ribonucleotides, or compounds produced synthetically (e.g. PNA as described in U.S. Patent No. 5,948,902 and the references cited therein) which can hybridize with naturally occurring nucleic acids in a sequence specific manner analogous to that of two naturally occurring nucleic acids.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length.

The term "polynucleotide" as used herein refers to single or double stranded polymer composed of nucleotide monomers of generally greater than 100 nucleotides in length.

The term "mRNA" means messenger RNA.

The term "array" means a substrate having at least one planar surface on which is immobilized a plurality of different probe nucleic acids.

Methods for generating mixtures of nucleic acids, e.g., oligonucleotide primers, are provided. In the subject methods, an array is employed as template to generate mixtures of nucleic acids via a template driven primer extension reaction. In preferred embodiments, each probe on the array employed in the subject methods comprises a constant domain and a variable domain, where the constant domain is further characterized by having at least a recognition domain, and optionally a functional and/or linker domain. Also provided are the arrays employed in the subject methods and kits for practicing the subject methods. The subject methods find use in a variety of applications, including the generation of target nucleic acids from an mRNA sample for use in hybridization assays, e.g., differential gene expression analysis. In further describing the subject invention, the subject methods will be described first, followed by a review of representative protocols in which the nucleic acid mixtures produced by the subject methods find use as well as a description of kits that find use in practicing the subject methods.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs.

As summarized above, the subject invention provides methods for generating mixtures of nucleic acids by a template driven primer extension protocol in which an array is employed as template. The mixture of nucleic acids produced by the subject methods is characterized by having a known composition. As such, at least the sequence of each individual or distinct nucleic acid in the mixture of differing sequence is known. In many embodiments, the relative amount or copy number of each distinct nucleic acid of differing sequence is known. Each nucleic acid present in the mixture at least includes a variable domain that serves to distinguish it from any other nucleic acid in the mixture, i.e., any other nucleic acid that does not have the identical sequence -- any nucleic acid that is not its copy. The variable domain, $S_{ij}$, is a nucleic acid that hybridizes under stringent conditions to gene i at location j and is capable of serving as a primer in reverse transcription beginning at base j. The number of different variable domains, $S_{ij}$, present in the mixture may vary, but is generally at least about 10, usually at least about 20 and more usually at least about 50, where the number may be as great as 25,000 or greater. In many embodiments, the number of different variable domains present in the mixture ranges from about 1,978 to 25,000, usually from about 4,200 to 8,400. In addition to the distinguishing variable domain, the constituent members of the mixture may all share one or more domains of common sequence, depending on the particular protocol employed to generate the mixture, as described in greater detail below.

In the subject methods, the first step is generally to provide an array, i.e., a substrate having a planar surface on which is immobilized a plurality of distinct nucleic acid probes, in which each probe sequence on the array includes a constant domain and a complement variable domain. This providing step may include either generating the array *de novo* or obtaining a pre-made array from a commercial source, where in either case the array will have the characteristics described below. Arrays of nucleic acids are known in the art, where representative arrays that may be modified to become arrays of the subject invention as described below, include those described in: 5,242,974; 5,384,261; 5,405,783; 5,412,087; 5,424,186; 5,429,807; 5,436,327; 5,445,934; 5,472,672; 5,527,681; 5,529,756; 5,545,531; 5,554,501; 5,556,752; 5,561,071; 5,599,695; 5,624,711; 5,639,603; 5,658,734; 5,795,714; WO 93/17126; WO 95/11995; WO 95/35505; EP 742 287; and EP 799 897; the disclosures of which are herein incorporated by reference.

As mentioned above, each distinct probe nucleic acid on the array includes a constant domain and a complement variable domain. The complement variable domain of each distinct probe has a sequence that is the complement of a variable or distinguishing domain found in a constituent member of the mixture of nucleic acids that is produced by the subject methods as described above, where by complement is meant that the variable and complement variable sequences hybridize under stringent conditions, e.g., at 50°C or higher and 0.1HSSC (15 mM sodium chloride/1.5 mM sodium citrate) or thermodynamically equivalent conditions. Thus, the array includes a plurality of distinct probes that differ from each other by complement variable domain, where the number of distinct probes on an array employed in the subject methods is typically at least 10, usually at least 20 and more usually at least 50, where the number may be as high as 25,000 or higher. In many embodiments, the number of distinct probes ranges from about 1,978 to 25,000, usually from about 4,200 to 8,400.

Because of the nature of the subject methods, as described below, each distinct complement variable domain will be represented in the nucleic acid mixture produced using the array, i.e., the complement of each distinct complement variable domain sequence will be found in the mixture of nucleic acids produced by the subject methods. For example, where an array has 10 different probes that differ by complement variable domain such that it has 10 different complement variable domains, i.e., $cV_{1-10}$, the nucleic acid mixture produced by the subject methods as described below will have 10 different or distinct nucleic acids, where each different nucleic acid sequence in the mixture includes a sequence that is the complement of one of $cV_{1-10}$, i.e., $V_{1-10}$.

The relative copy number of each probe on the array may or may not be selected to "normalize" the nucleic acid mixture made with the array with respect to the mRNA sample with which it is to be used. For example, if the array is to be used to make a nucleic acid mixture that has a 10-fold increase in the copy number of target that hybridizes to a rare mRNA, the copy number of the corresponding (e.g. identical or complementary) probe on the array can be appropriately increased relative to other probes that correspond to less rare mRNA species in the mRNA sample. In many embodiments, the complement variable domain is a domain that has a sequence that is chosen to hybridize under stringent conditions to a sequence of interest found in a particular mRNA. In many embodiments, the complement variable sequence has a sequence that is denoted as $cS_{ij}$, where c stands for complement and $S_{ij}$ is a nucleic acid that primes reverse transcription of a gene i beginning at base j. Thus, in many embodiments of the invention, the complement variable domain of each probe is the complement of a nucleic acid that is capable of hybridizing to a different gene of interest i at location or base j and acting as a primer under reverse transcription conditions. For example, where 10 different genes, i.e., genes 1 to 10 are represented on the array and the sequence of interest for each gene begins at base number 50, 60, 70, 80, 90, 100, 110, 120, 130 and 140, respectively (counting from the 5' end of the mRNA molecule), and each complement variable domain is 20 bases long, the complement variable domains of each distinct probe on the array, i.e., $cV_1$ to $V_{10}$, will be as follows:

| Variable Domain | Sequence |
| --- | --- |
| $cV_1$ | Sequence that hybridizes under stringent conditions to bases 50 to 30 of gene 1 |
| $cV_2$ | Sequence that hybridizes under stringent conditions to bases 60 to 40 of gene 2 |
| $cV_3$ | Sequence that hybridizes under stringent conditions to bases 70 to 50 of gene 3 |
| $cV_4$ | Sequence that hybridizes under stringent conditions to bases 80 to 60 of gene 4 |
| $cV_5$ | Sequence that hybridizes under stringent conditions to bases 90 to 70 of gene 5 |
| $cV_6$ | Sequence that hybridizes under stringent conditions to bases 100 to 80 of gene 6 |
| $cV_7$ | Sequence that hybridizes under stringent conditions to bases 110 to 90 of gene 7 |
| $cV_8$ | Sequence that hybridizes under stringent conditions to bases 120 to 100 of gene 8 |
| $cV_9$ | Sequence that hybridizes under stringent conditions to bases 130 to 110 of gene 9 |
| $cV_{10}$ | Sequence that hybridizes under stringent conditions to bases 140 to 120 of gene 10 |

While the length of the complement variable domain in the specific example provided above is 20 bases or residues, i.e., 20 nt, the length may vary considerably and will be chosen based on the desired length of the resultant nucleic acids in the to be produced mixture within the synthesis constraints of the subject method. Generally, the length of the complement variable domain will range from about 15 to 40, usually from about 15 to 30 and more usually from about 20 to 25 nt.

As mentioned above, in addition to the unique complement variable domain, each probe nucleic acid present on the array includes a common or shared constant domain 3' of the complement variable domain. This constant domain typically ranges in length from about 20 to 50, usually from about 20 to 45 and more usually from about 25 to 40 nt. The constant domain typically comprises at least one of the following constant sub-domains: a functional domain; a recognition domain and a linker domain. In many embodiments, each probe contains at least a recognition sub-domain, and optionally a functional domain and/or a linker domain. These constant sub-domains may be grouped together on the probe or separated so as to flank the variable domain of the probe. As such, in certain embodiments these sub-domains are generally arranged in the order of functional domain, recognition domain and linker domain going from the 5' to the 3' end of the probe sequence, such that the linker domain is at the 3' probe terminus and is attached, either directly or indirectly, to the substrate surface of the array. In yet other embodiments, one or more of the domains, e.g., the functional sub-domain, may be present on the 5' end of the variable domain.

The optional functional sub-domain is generally a sequence that imparts or contributes some function to a duplex nucleic acid in which it is present. Functional domains of interest include: polymerase promoter sites, e.g., T3 or T7 RNA polymerase promoter sites, sequences unique with respect to the intended target organism for the array experiment (i.e. unique priming sites) and the like. The length of this functional domain typically ranges from about 10 nt to 40 nt, usually from about 20 nt to 30 nt.

The recognition sequence of the constant domain is typically a sequence that, when present in duplex format, is recognized and cleaved by a restriction endonuclease. A large number of restriction endonucleases are known to those of skill in the art. Specific restriction endonuclease recognized sites of interest that may make up the subject recognition sequence include, but are not limited to: Hinc II and the like. Generally, the length of the recognition domain ranges from about 4 nt to 8 nt, usually from about 5 nt to 6 nt

The linker sub-domain of the subject constant domains is optional. The linker domain may be any convenient sequence, including random sequence or a non-polynucleotide chemical linker (e.g. an ethylene glycol-based polyether oligomer), where the sole purpose of the linker domain is to project the other domains of the probe away from the substrate surface. Generally, the linker domain if present, has a length ranging from about 1 to 20, usually from about 1 to 15 and more usually from about 1 to 10, including 5 to 10 nt.

In many, though not all, embodiments, each surface bound probe on the array employed in the subject methods is described by the following formula:

$$\text{surface-3'-L-R-F-cV-5'}$$

wherein:

L is the optional linking domain;
R is the recognition domain;
F is the functional domain; and
cV is the complement variable domain, i.e., the complement of the variable domain, $cS_{ij}$, of the nucleic acid produced by the subject methods to which it hybridizes under stringent conditions;

where each of these elements are as described above.

As mentioned above, the subject arrays are provided by any convenient means, including obtaining them from a commercial source or by synthesizing them *de novo.* To synthesize the arrays employed in the subject methods, the first step is generally to determine the nature of the mixture of nucleic acids that is to be produced using the subject array according to the subject methods. In those embodiments where the nucleic acid mixture is to be employed as gene specific primer in the generation of target nucleic acid, as described in greater detail below, the first step is to identify those genes that are to be represented by a primer in the primer mixture, i.e., those specific mRNAs potentially present in the experimental samples which are to have primers in the mixture that are capable of hybridizing to them under stringent conditions. Following identification of these genes, the specific region, i.e. stretch or domain, of each mRNA to which the primer is to hybridize is then identified. These specific domains or regions may be identified using any convenient protocol and set of selection criteria, where of interest in many embodiments is the use of the algorithm and selection methods based thereon described in U.S. Patent 6,251,588, the disclosure of which is herein incorporated by reference. As such, a plurality of different sequences of interest will be identified, wherein each sequence is described by the formula $S_{ij}$,

where i is the gene of interest and j is the specific base at which the sequence starts, as described above. Following identification of each variable or $S_{ij}$ sequence as described above, a probe sequence for each different variable or $S_{ij}$ sequence is identified, where the probe sequence has the following sequence in many embodiments:

$$\text{3'-L-R-F-cV-5'}$$

wherein:

L is the linking domain;
R is the recognition domain;
F is the functional domain; and
cV is the complement of the variable domain, i.e., $cS_{ij}$;

where each of these elements are as defined above and each of the probes varies only in terms of its cV domain.

Following identification of the probe sequences as defined above, an array is produced in which each of the probe sequences of the identified set is present. The array may be produced using any convenient protocol, where suitable protocols include both synthesis of the complement probe followed by deposition onto a substrate surface, as well as synthesis of the probe directly on the substrate surface. Representative protocols for array synthesis are described in: 5,242,974; 5,384,261; 5,405,783; 5,412,087; 5,424,186; 5,429,807; 5,436,327; 5,445,934; 5,472,672; 5,527,681; 5,529,756; 5,545,531; 5,554,501; 5,556,752; 5,561,071; 5,599,695; 5,624,711; 5,639,603; 5,658,734; 5,795,714; WO 93/17126; WO 95/11995; WO 95/35505; EP 742 287; and EP 799 897; the disclosures of which are herein incorporated by reference.

Following provision of the array employed in the subject methods, as described above, the next step is to contact the array with universal primer under hybridization conditions sufficient to produce a template array that includes a plurality of overhang comprising duplex nucleic acids on its surface, where the overhang is made up of the complement variable domain of each probe of the array. The universal primer is capable of hybridizing to the constant domain, or at least a portion thereof (e.g., at least that portion immediately 3' of the complement variable domain). The universal primer has a length that is sufficient to prime template driven primer extension, where the length of the universal primer generally ranges from about 10 to 45 nt, usually from about 15 to 35 nt and more usually from about 20 to 30 nt. In many embodiments, the universal primer is the complement of the recognition and/or functional sub-domains of the constant domain of each probe on the array. As such, in many embodiments the universal primer employed has a sequence described by the formula:

$$5'\text{-cR-cF-3'}$$

wherein:

cR is the complement of the recognition domain; and
cF is the complement of the functional domain.

As mentioned above, the template array produced by this method is an array of duplex probe molecules made up of a first nucleic acid having a constant and complement variable domain and a second nucleic acid which is the universal primer and is hybridized to the constant domain (or at least that portion of the constant domain that is 3' of the variable domain complement). As such, the array produced by this step is an array of overhang comprising duplex nucleic acid, typically DNA, molecules, where the overhang is made up of the complement variable domain of each probe on the array.

This template array of overhang comprising duplex probes is then subjected to primer extension reaction conditions sufficient to produce the desired mixture of nucleic acids. The specific primer extension reaction conditions to which the template array of overhang comprising duplex nucleic acids is subjected may vary depending on the particular protocol used and/or the specific nature of the nucleic acid mixture to be produced therefrom. Specific primer extension reaction conditions of interest include, but are not limited to: linear PCR (Polymerase Chain Reaction); strand displacement amplification; and *in vitro* transcription. Each of these specific primer extension reaction conditions is now reviewed in greater detail.

Where the template array is subjected to linear PCR conditions, the array is contacted in an aqueous reaction mixture with a source of DNA polymerase, dNTPs and any other desired or requisite primer extension reagents under conditions sufficient to produce linearly amplified amounts of nucleic acids, e.g., under thermal cycling conditions. As such, the polymerase employed in the subject methods is generally, though not necessarily (e.g., where new polymerase is added after each cycle) a thermostable polymerase. A variety of thermostable polymerases are known to those of skill in the art, where representative polymerases include, but are not limited to: Taq polymerase, Vent® polymerase, Pfu polymerase and the like. The amount of polymerase present in the reaction mixture may vary but is sufficient to provide for the requisite amount of polymerase activity, where the specific amount employed may be readily determined by those of skill in the art. Also present in the reaction mixture is a collection of the four dNTPs, i.e., dATP, dCTP, dGTP and dTTP. The dNTPs may be present in varying or equimolar amounts, where the amount of each dNTP typically ranges from about 10 μM to 10 mM, usually from about 100 μM to 300 μM. Other reagents that may be present in the reaction mixture include: monovalent cations (e.g. Na$^+$), divalent cations (e.g. Mg$^{++}$), buffers (e.g. Tris), surfactants (e.g. Triton X-100) and the like. In this linear PCR embodiment of the subject methods, the reaction mixture is subjected to thermal cycling conditions in which the temperature of the reaction mixture is cycled through an annealing, primer extension and dissociation temperatures in a manner that results in the production of linearly amplified amounts of nucleic acid for each different sequence probe on the template array. The annealing temperature typically ranges from about 50°C to 80°C, usually from about 60°C to 75°C and is maintained for period of time ranging from about 10 sec. to 10 min., usually from about 30 sec. to 2 min. The primer extension temperature typically ranges from about 55°C to 75°C, usually from about 60°C to 70°C and is maintained for period of time ranging from about 30 sec. to 10 min., usually from about 1 min. to 5 min.. The dissociation temperature typically ranges from about 80°C to 99°C, usually from about 90°C to 95°C and is maintained for period of time ranging from about 1 sec. to 2 min., usually from about 30 sec. to 1 min.

In strand displacement amplification, the array of overhang comprising duplex nucleic acids is employed as primed template in linear amplification variations of the exponential amplification protocols described in Walker et al., Nucleic Acids Res. (1992) 20:1691-1696 and Walker et al., Proc. Nat'l Acad. Sci. USA (1992) 89:392-396; as well as in U.S. Patent No. 5,648,211; the disclosure of which is herein incorporated by reference. Briefly, isothermal linear amplification is achieved as follows. Following production of the array of overhang comprising duplex nucleic acids, the template array is subjected to a cycle of strand nicking of the universal primer after sequence cR, typically by using a restriction endonuclease. Generally, the template strand or probe sequence is protected via an appropriately placed phosphorthioate linkage in the surface-bound template strand. Extension of the 3' end exposed by the nick is then allowed to proceed by using a DNA polymerase that lacks a 5'→3' exonuclease activity but possesses a strand displacement activity, e.g., Klenow fragment. Each cycle in this protocol releases a nucleic acid molecule which has the formula: 5'-cF-Sij-3'. In certain variants of this method, nicking may be achieved by making R a half-site for a restriction endonuclease that exhibits single-strand cleavage activity, or by employing a nicking endonuclease, such as N.BstNBI, and the like.

In yet other embodiments, the subject template array of duplex nucleic acids is employed in an *in vitro* transcription method. In this embodiment, the template array is modified from that described above to be of the following formula:

$$\text{(surface)-L-R-(C)Sij-F-5'}$$

wherein:

L and R are as defined above;
F is an RNA polymerase promoter, e.g., T3 or T7 promoter; and
(C) Sij is Sij modified to end in a C residue.

The universal primer employed with this array has the formula 5'-cR-3'. When the template array is contacted with NTPs, T3 or T7 polymerase and the appropriate transcription buffer, ribonucleic acids of the formula 5'-(rG)rcSij-rcF-3' are produced, where r stands for ribonucleotide. By contacting this resultant mixture of ribonucleic acids with the DNA primer 5'-F-3' and a reverse transcriptase, a mixture of deoxyribonucleic acids suitable for use as primer in target generation protocols is produced.

The subject template arrays may also be used in other nucleic acid primer extension generation protocols—the above being merely representative of the protocols in which the subject template arrays find use.

The above described array template based primer extension generation methods result in the production of a mixture of nucleic acids, typically a mixture of deoxyribonucleic acids, where each of the different complement variable domains of the template array is represented in the mixture, i.e., there is at least one nucleic acid in the mixture that has a variable domain that hybridizes under stringent conditions to each different complement variable domain present on the array. The length of each of the nucleic acids present in the resultant mixture typically ranges from about 20 to 60 nt, usually from about 25 to 55 nt and more usually from about 30 to 50 nt. Because of the manner in which the subject mixtures of nucleic acids are produced, the resultant mixtures of nucleic acids may be viewed as mixtures of gene specific primers, where the gene specific primers are specific for each of the different genes represented on the template array employed in the production of the nucleic acid mixture. In certain embodiments, the mixture may be "normalized" with respect to a given mRNA population, as described above.

The nucleic acid mixtures produced by the subject methods find use in a variety of different applications, and are particularly suited for use as primers in the generation of target nucleic acids, e.g., for array based differential gene expression analysis applications. Where the subject nucleic acids mixtures are used as primers for target generation in gene expression analyses, the first step is to generate a population of target nucleic acids from an initial mRNA source or sample. By target nucleic acid is meant a nucleic acid that has a sequence, e.g., $S_{ij}$, which is either the same as, or complementary to, the sequence of an mRNA found in an initial sample, where the target may be DNA or RNA and be present in amplified amounts as compared to the initial amount of mRNA, depending on the particular target generation protocol that is employed.

In the subject methods, the target or image nucleic acids are produced from the subject nucleic acid mixtures generally through enzymatic generation protocols. Specifically, the target nucleic acids are typically produced using template dependent polymerization protocols and an initial mRNA source. The initial mRNA source may be present in a variety of different samples, where the sample will typically be derived from a physiological source. The physiological source may be derived from a variety of eukaryotic or prokaryotic sources, with physiological sources of interest including sources derived from single-celled organisms such as yeast and multicellular organisms, including plants and animals, particularly mammals,

where the physiological sources from multicellular organisms may be derived from particular organs or tissues of the multicellular organism, or from isolated cells derived therefrom. In obtaining the sample of RNA to be analyzed from the physiological source from which it is derived, the physiological source may be subjected to a number of different processing steps,

where such processing steps might include tissue homogenization, cell isolation and cytoplasm extraction, nucleic acid extraction and the like, where such processing steps are known to those of skill in the art. Methods of isolating RNA from cells, tissues, organs or whole organisms are known to those of skill in the art and are described in Maniatis *et al*. (1989), Molecular Cloning: A Laboratory Manual 2d Ed. (Cold Spring Harbor Press).

A number of different enzymatic protocols for generating image or target nucleic acids from an initial mRNA sample are known and continue to be developed. Any convenient protocol may be employed, where the particular protocol employed depends, at least in part, on a number of factors, including: whether one wants to generate amplified amounts of target or image nucleic acid; whether one wants to generate geometrically or linearly amplified amounts of target nucleic acid; whether bias in the amount of target can be tolerated, etc. A common feature of the protocols that find use in preparing the image or target nucleic acids of the subject invention is the use of the subject nucleic acid mixtures produced using array-based template protocols described above as primer.

A number of nucleic acid amplification methods can be employed to generate the target nucleic acid from an initial mRNA source, where these methods can employ the subject nucleic acid mixtures as primer. Such methods include the "polymerase chain reaction" (PCR) as described in United States Patent Number 4,683,195, the disclosure of which is herein incorporated by reference, and a number of transcription-based exponential amplification methods, such as those described in U.S. Patent Nos. 5,130,238; 5,399,491; and 5,437,990; the disclosures of which are herein incorporated by reference. Each of these methods uses primer-dependent nucleic acid synthesis to generate a DNA or RNA product, which serves as a template for subsequent rounds of primer-dependent nucleic acid synthesis. Each process uses (at least) two primer sequences complementary to different strands of a desired nucleic acid sequence and results in an exponential increase in the number of copies of the target sequence.

Alternatively, amplification methods that utilize a single primer may be employed to generate target or image nucleic acids from an initial mRNA sample, where the subject nucleic acid mixtures are employed as primer. See e.g. U.S. Patent Nos. 5,554,516; and 5,716,785; the disclosures of which are herein incorporated by reference. The methods reported in these patents utilize a single primer containing an RNA polymerase promoter sequence and a sequence complementary to the 3'-end of the desired nucleic acid target sequence(s) ("promoter-primer"). In both methods, the promoter-primer is added under conditions where it hybridizes to the target sequence(s) and is converted to a substrate for RNA polymerase. In both methods, the substrate intermediate is recognized by RNA polymerase, which produces multiple copies of RNA complementary to the target sequence(s) ("cRNA").

Whatever process is employed to generate the target nucleic acid, where representative protocols have been provided immediately above, the process may be modified to include the use of chemical analogs of nucleotides that have been modified to include a label moiety, e.g., an organic fluorophore, an isotopic label, a capture ligand, e.g., biotin, etc. As a result, the target nucleic acids produced using the subject nucleic acid mixtures as primers often are labeled, either directly or indirectly, for use in subsequent hybridization assays.

The above target generation protocols are merely representative and by no means inclusive of all of the different types of protocols in which the subject nucleic acid mixtures find use as primers.

The resultant populations of target nucleic acids find use as, *inter alia*, target in hybridization assays, such as gene expression analysis applications. Gene expression analysis protocols are well known to those of skill in the art, and the populations of target nucleic acids produced by the subject methods find use in many, if not all, of these protocols. In gene expression analysis protocols using the subject populations of labeled target, the population of labeled target is typically contacted with a population of probe nucleic acids, e.g., on an array, under hybridization conditions, usually stringent hybridization conditions. The array may be the same array that is used as the template array or a different array. Following hybridization, non-bound target is removed or separated from the probe, e.g., by washing. Washing results in a pattern of hybridized target, which may be read using any convenient protocol, e.g., with a fluorescent scanner device. From this pattern, information regarding the mRNA expression profile in the initial mRNA sample from which the target population was produced may be readily derived or deduced.

In certain embodiments, the subject methods include a step of transmitting data from at least one of the detecting and deriving steps, as described above, to a remote location. By "remote location" is meant a location other than the location at the which the array is present and hybridization occur. For example, a remote location could be another location (e.g. office, lab, etc.) in the same city, another location in a different city, another location in a different state, another location in a different country, etc. The data may be transmitted to the remote location for further evaluation and/or use. Any convenient telecommunications means may be employed for transmitting the data, e.g., facsimile, modem, internet, etc.

Also provided by the subject invention are kits for use in preparing the subject target populations of nucleic acids. The kits may comprise containers, each with one or more of the various reagents (typically in concentrated form) utilized in the methods, including, for example, buffers, dNTPs, reverse transcriptase, etc., where the kits will at least include a sufficient amount of universal primer, e.g., an amount ranging from about 25 pmol to 25 μmol. In addition, the subject kits may include an array of single stranded probe nucleic acids (or a means for producing the same) wherein each probe has a constant region and complement variable region, as described above. Where the kit has a means for producing the template array, the kit typically includes a substrate having a planar surface, and one or more reagents necessary for synthesis of the probes, which may vary depending on the nature of the protocol to be used to generate the array. The kits may further include reagents necessary for producing labeled target nucleic acids, where such reagents may include reverse transcriptase, labeled dNTPs, etc. A set of instructions will also typically be included, where the instructions may be associated with a package insert and/or the packaging of the kit or the components thereof.

The following examples are offered by way of illustration and not by way of limitation.

EXPERIMENTAL

Example

In order to demonstrate the feasibility of using an oligonucleotide array as a template for enzymatic polynucleotide synthesis, the following experiment was performed:

1. An *in situ* oligonucleotide array was manufactured; the array contained 8455 (89 x 95) features (~100 μm diameter) with the following sequence:

5'-

CTTTCTTGGATCAACCCGCTCAATGCTCCCTATAGTGAGTCGTATTACAATTCATTT

TTT-surface (SEQ ID NO:01)

In the above sequence, the large dash underlines indicate the unique sequence $cS_{ij}$, the small dashes indicate the recognition/functional sequence *F-R* (in this case, a T7 RNA polymerase promoter) and the continuous underline indicates a linker sequence *Q.*

2. The array was hybridized for 1 hour at 60°C to the following oligonucleotide (PT7, 250 nM)

3'-GATATCACTCAGCATAATGTTAAGTA-5' (SEQ ID NO:02)

i.e. the complementary strand of the T7 promoter portion of the oligonucleotide on the surface. The purpose of this treatment was to produce a double-stranded T7 promoter, which is necessary for T7 RNA polymerase activity (note that a double-stranded template strand is not necessary; a 5'-overhanging single-stranded template is known to be sufficient).

3. The array was washed briefly with ice-cold water (to remove salts from the hybridization buffer) and blown dry with nitrogen. The hybridization chamber was reassembled and filled with a transcription mixture (250 μl) containing T7 transcription buffer (including NTP's), T7 RNA polymerase, 1% Triton X-100 and the oligonucleotide of step 2 (250 nM). The assembly was incubated overnight at 40°C. An identical positive control array was also incubated in contact with the same transcription mixture, with a soluble version of the array-bound oligonucleotide of step 1 added (HCV 185; 250 nM). Finally, a second positive control mixture was incubated in a PCR tube.

4. The transcription mixtures were removed from the experimental and positive control arrays. Half of each array mixture was concentrated >10x using a Microcon-3 ultrafiltration concentrator.

5. The various samples were analyzed on a 15% polyacrylamide/4M urea gel, stained with ethidium bromide and visualized by fluorescence. The results are provided in Fig. 1.

The results provided in Fig. 1 clearly show visible transcript in the concentrated experimental array sample (lane 2). Separate negative control experiments demonstrated that reactions which omitted the complementary oligonucleotide PT7 or the T7 RNA polymerase did not produce visible bands on a similar gel (data not shown). Microcon concentration of ~80 μl of 250 nM PT7 oligo also failed to yield a visible band on a similar gel (data not shown). Thus, the observed gel pattern is dependent upon the presence of T7 RNA polymerase and a double-stranded T7 promoter, and is not due to the added oligonucleotide PT7. Furthermore, the chief product of transcription from an array-bound template displays the same gel migration rate as the chief product of positive-control transcription reactions. The most likely explanation for the observed data is that we have reduced to practice the T7 RNA polymerase version of enzymatic oligonucleotide production from an array template.

It is evident that the subject invention provides a number of advantages over current target nucleic acid generation protocols. These advantages include the provision of an economical and rapid synthesis method for custom primer mixtures that are particularly suited for use in target generation for use with the nucleic acid arrays. Using the subject methods leads to increased specificity in microarray based assays. Using the subject methods, one can develop microarray based assays in which the microarray is customized to be sensitive or insensitive to various splicing variants of different genes of interest, even where the splicing variant is present proximal to the 5' end of the coding sequence. Allele specific mRNA profiling is possible with the subject methods by picking the variable region so that the 3'-end of the primer produced hybridizes at a base where the two alleles differ. In addition, the subject methods can be employed to easily produce normalized target nucleic acid mixtures. Accordingly, the invention represents a significant contribution to the art.

All publications and patent applications cited in this specification are herein incorporated by reference as if each individual publication or patent application were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

This invention also provides an array comprising a plurality of distinct single-stranded probe nucleic acids immobilized on a surface of a substrate, wherein each of said single-stranded probe nucleic acids is described by the formula:

surface-L-R-F-cV-5'

wherein:

L is an optional linking domain;
R is a recognition domain;
F is a functional domain; and
V is a variable domain;

wherein only said variable domain V is different for each distinct single-stranded probe nucleic acid of said array.

This invention also provides a kit comprising:

(a) universal primer; and
(b) an array of probe nucleic acids or a means for producing the same.

This invention also provides a method for producing a mixture of nucleic acids, said method comprising:

(a) providing an array of distinct single-stranded probe nucleic acids of differing sequence where each distinct probe present on said array comprises a constant domain and a complement variable domain;
(b) contacting said array of single-stranded probe nucleic acids with nucleic acids complementary to said constant domain under hybridization conditions, whereby a template array of overhang-comprising duplex nucleic acids is produced, wherein each overhang-comprising duplex nucleic acid of said array comprises a double-stranded constant region and a single-stranded variable region overhang; and
(c) subjecting said template array of overhang-comprising duplex nucleic acids to primer extension reaction conditions under conditions sufficient to produce said mixture of nucleic acids;

whereby said mixture of single-stranded primer nucleic acids.


**Claims**

1.  A method for producing a mixture of nucleic acids, said method comprising:

    (a) providing an array of distinct single-stranded probe nucleic acids of differing sequence where each distinct probe present on said array comprises a constant domain and a complement variable domain, wherein the constant domain comprises a sequence that, when present in duplex format, is recognized and cleaved by a restriction endonuclease;
    (b) contacting said array of single-stranded probe nucleic acids with nucleic acids complementary to said constant domain under hybridization conditions, whereby a template array of overhang-comprising duplex nucleic acids is produced, wherein each overhang-comprising duplex nucleic acid of said array comprises a double-stranded constant region and a single-stranded variable region overhang; and
    (c) contracting said array with a restriction endonuclease to produce a mixture of cleaved nucleic acids.

2.  A method as claimed in claim 1, further comprising subjecting said overhang-comprising duplex nucleic acids to linear PCR, strand displacement amplification or an *in vitro* transcription protocol.

3.  A method as claimed in Claim 1 or 2, wherein said mixture of nucleic acids is a mixture of deoxyribo-oligonucleotides.

4.  A method as claimed in Claim 1, 2 or 3, wherein the sequence of each distinct nucleic acid in the mixture is known.

5.  The method according to any of Claims 1 to 4, wherein each distinct surface immobilized single-stranded probe present on said array is described by the formula:

surface-L-R-F-cV-5'

wherein:

L is a linking domain;
R is a recognition domain;
F is a functional domain; and
cV is a complement domain having a sequence that hybridizes under stringent conditions to a variable domain of one of said distinct oligonucleotides of said plurality; and

said nucleic acids complementary to said constant domain are of the formula:

5'-cR-cF-3'

wherein:

cR is the complement of R; and
cF is the complement of F.

6.  A method as claimed in Claim 5, wherein said linker domain has a length of 0 to 10 bases.

7.  A method as claimed in Claim 5 or 6, wherein said functional domain is an RNA polymerase promoter domain.

8.  A method as claimed in Claim 5, 6 or 7, wherein said recognition domain is a recognized by a restriction endonuclease.

9.  A method of making a population of target nucleic acids from an initial mRNA sample, said method comprising:

    (a) generating a mixture of nucleic acids according to the method of any of Claims 1 to 8; and
    (b) employing said mixture of nucleic acids as primers in a target generation step in which target nucleic acids are produced from said mRNA sample;

    whereby said population of target nucleic acids is produced.

10. A hybridization assay comprising the steps of:

    (a) generating a set of target nucleic acids according to the method of Claim 9;
    (b) contacting said set of target nucleic acids with an array of probe nucleic acids under hybridization conditions; and
    (c) detecting the presence of target nucleic acids hybridized to probe nucleic acids of said array.

FIGURE 1

L= 77KB RNA ladder
1= Experimental array 2 (annealed with PT7)
2= Concentrated experimental array 2
3= Positive control array 4 (annealed with PT7 AND PT7/HVC185
complex in mix)
4= Concentrated positive control array 4
5= Positive control tube


SEQUENCE LISTING

```
<110>  Agilent Technologies, Inc.

<120>  Array based methods for synthesizing nucleic acid mixtures

<130>  IT/KE/N17256

<150>  US 09/628,472
<151>  2000-07-31

<150>  EP 01306573.5
<151>  2001-07-31

<160>  2

<170>  PatentIn version 3.3

<210>  1
<211>  60
<212>  DNA
<213>  artificial sequence

<220>
<223>  synthetic probe

<400>  1
ctttcttgga tcaacccgct caatgctccc tatagtgagt cgtattacaa ttcatttttt      60


<210>  2
<211>  26
<212>  DNA
<213>  artificial sequence

<220>
<223>  synthetic probe

<400>  2
gatatcactc agcataatgt taagta                                           26
```